# EUROPEAN PATENT APPLICATION

(11) **EP 4 443 439 A1**
(43) Date of publication of application: **09.10.2024**
(21) Application number: 24165392.2
(22) Date of filing: 22.03.2024
(51) Int. Cl.: G16H 20/30, A61B 5/11, A61B 5/00, G16H 50/30

(54) **A METHOD AND SYSTEM FOR ESTIMATING A FUTURE PERFORMANCE**

(30) Priority: 06.04.2023 FI 20235397
(71) Applicant: Summa Finland Oy, 90620 Oulu (FI)
(72) Inventor: Nissilä, Juuso, 90230 Oulu (FI); Huovinen, Jukka, 33400 Tampere (FI); Kinnunen, Tommi, 00190 Helsinki (FI); Juurikka, Aleksi, 91300 Oulu (FI); Mäkinen, Marko, 90500 Oulu (FI)
(74) Representative: Moosedog Oy

(57) **Abstract**

Disclosed is a method for estimating a future value of at least one performance parameter of a first user. The method comprises measuring, for first user, a first set of values of at least one molecular biomarker and a first set of corresponding values of at least one performance parameter; determining a first correlation between first set of values of at least one molecular biomarker and first set of corresponding values of at least one performance parameter; using determined first correlation to generate a first performance prediction function for first user that indicates at least one performance parameter as a function of at least one molecular biomarker; measuring, for first user, a prediction request value of at least one molecular biomarker; and using first performance prediction function to estimate future value of at least one performance parameter, based on prediction request value of at least one molecular biomarker.

## Description

### TECHNICAL FIELD

The present disclosure relates to methods for estimating future values of at least one performance parameter of a first user. The present disclosure also relates to systems for estimating future values of at least one performance parameter of a first user. The present disclosure further relates to methods for selecting a first set of users from amongst a plurality of users.

### BACKGROUND

Generally, across a wide range of sports and physical activities, such as running, walking, high jump, long jump, skiing, weightlifting, and the like, athletes need to constantly track their respective performance by tracking certain measurable parameters. Notably, tracking such measurable parameters help the athletes in planning their training schedules and analysing their chances of success in future competitions.

However, conventional solutions fail to track certain measurable parameters with respect to functional biomarkers of the athlete. As a result, the conventional solutions fail to provide an efficient solution to accurately and precisely predict the future performance of the respective athlete, due to a lack of good correlation between measurable parameters and functional biomarkers of the athlete.

In an example, measurable parameters of athletes can be measured using timer to find out speed of running. Functional biomarker related to heart rate can be measured using for example heart rate monitors. The heart rate can be used further to derive one commonly used indication, related to possible performance i.e. heart rate variability (HRV). Said functional biomarker is typically measured when the athlete trains and used to plan training schedules and amounts for the athlete. Alternatively, other parameters such as fitness levels for the athlete may be measured when the athlete trains to plan the training schedules for the athlete. However, said functional biomarker fail to predict a future success in the competition.

Therefore, in light of the foregoing discussion, there exists a need to overcome the aforementioned drawbacks.

### SUMMARY

The aim of the present disclosure is to provide a method for estimating a future value of at least one performance parameter of a first user. The aim of the disclosure is achieved by a method which accurately and precisely estimates a future performance of the first user based on at least one molecular biomarker of the first user as defined in the appended independent claims to which reference is made to. Advantageous features are set out in the appended dependent claims.

The aim of the present disclosure is to provide a system for estimating a future value of at least one performance parameter of a first user. The aim of the disclosure is achieved by a system which accurately and precisely measures at least one molecular biomarker of the first user based on which a future performance of the first user is estimated as defined in the appended independent claims to which reference is made to. Advantageous features are set out in the appended dependent claims.

The aim of the present disclosure is to provide a method for selecting a first set of users from amongst a plurality of users. The aim of the disclosure is achieved by a method which accurately identifies top performers in performing the first type of physical activity as defined in the appended independent claims to which reference is made to. Advantageous features are set out in the appended dependent claims.

Additional aspects, advantages, features and objects of the present disclosure would be made apparent from the drawings and the detailed description of the illustrative embodiments construed in conjunction with the appended claims that follow.

It will be appreciated that features of the present disclosure are susceptible to being combined in various combinations without departing from the scope of the present disclosure as defined by the appended claims.

### BRIEF DESCRIPTION OF THE DRAWINGS

The summary above, as well as the following detailed description of illustrative embodiments, is better understood when read in conjunction with the appended drawings. For the purpose of illustrating the present disclosure, exemplary constructions of the disclosure are shown in the drawings. However, the present disclosure is not limited to specific methods and instrumentalities disclosed herein. Moreover, those skilled in the art will understand that the drawings are not to scale. Wherever possible, like elements have been indicated by identical numbers.

Embodiments of the present disclosure will now be described, by way of example only, with reference to the following diagrams wherein:
FIG. 1 illustrates a flowchart depicting steps of a method for estimating a future value of at least one performance parameter of a first user, in accordance with an embodiment of the present disclosure;
FIG. 2 illustrates a block diagram of a system for estimating a future value of at least one performance parameter of a first user, in accordance with an embodiment of the present disclosure;
FIG. 3 illustrates a flowchart depicting steps of a method for selecting a first set of users from amongst a plurality of users, in accordance with an embodiment of the present disclosure;
FIGs. 4-9 illustrate graphical representations depicting a respective performance parameter with respect to a respective molecular or functional biomarker, in accordance with an embodiment of the present disclosure; and
FIG. 10 illustrates a graphical representation depicting a first biomarker with respect to a second biomarker, in accordance with an embodiment of the present disclosure.

### DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognize that other embodiments for carrying out or practising the present disclosure are also possible.

Throughout the description and claims of this specification, the words "*comprise*" and "*contain*" and variations of the words, for example "*comprising*" and "*comprises*", mean "*including but not limited to*", and do not exclude other components, integers or steps. Moreover, the singular encompasses the plural unless the context otherwise requires: in particular, where the indefinite article is used, the specification is to be understood as contemplating plurality as well as singularity, unless the context requires otherwise.

In a first aspect, an embodiment of the present disclosure provides a method for estimating a future value of at least one performance parameter of a first user, the method comprising:
measuring, for the first user, a first set of values of at least one molecular biomarker and a first set of corresponding values of the at least one performance parameter;
determining a first correlation between the first set of values of the at least one molecular biomarker and the first set of corresponding values of the at least one performance parameter;
using the determined first correlation to generate a first performance prediction function for the first user that indicates the at least one performance parameter as a function of the at least one molecular biomarker;
measuring, for the first user, a prediction request value of the at least one molecular biomarker; and
using the first performance prediction function to estimate the future value of the at least one performance parameter, based on the prediction request value of the at least one molecular biomarker.

In a second aspect, an embodiment of the present disclosure provides a system for estimating a future value of at least one performance parameter of a first user, the system comprising
a measurement arrangement for measuring a first set of values of at least one molecular biomarker for a first user;
a measurement device that is to be employed for measuring a first set of corresponding values of the at least one performance parameter for the first user for a first type of physical activity; and
a server configured to:
   receive the first set of values of the at least one molecular biomarker and the first set of corresponding values of the at least one performance parameter; and
   generate, using the received first set of values of the at least one molecular biomarker and the first set of corresponding values of the at least one performance parameter, a first performance prediction function for the first user that indicates the at least one performance parameter as a function of the at least one molecular biomarker.

In a third aspect, an embodiment of the present disclosure provides a method for selecting a first set of users from amongst a plurality of users, the method comprising:
generating respective performance prediction functions for the plurality of users;
receiving respective prediction request values of at least one molecular biomarker for the plurality of users;
using, the respective prediction request values of the at least one molecular biomarker as respective inputs to the respective performance prediction functions for the plurality of users, for estimating respective future values of at least one performance parameter for the plurality of users; and
selecting the first set of users from amongst the plurality of users, based on the respective future values of the at least one performance parameter for the plurality of the users, wherein the first set of users comprise users having N highest values out of the future values of the at least one performance parameter, wherein the first set of users comprises N users.

The present disclosure provides the aforementioned method, and the aforementioned system, for estimating a future value of at least one performance parameter of a first user. The method and the system enables using at least one molecular biomarker of the first user that has a good correlation with the at least one performance parameter of the first user. Moreover, based on the good correlation between the at least one molecular biomarker and the at least one performance parameter, the method and the system accurately and precisely estimates the future value of the at least one performance parameter of the first user. This is achieved by providing a prediction request value of at least one molecular biomarker as input to the correlation of the molecular biomarker and performance. The present disclosure also provides the aforementioned method, for selecting a first set of users from amongst a plurality of users. The method enables in efficiently identifying top performers that are performing the first type of physical activity in the form of the first set of users from amongst the plurality of users.

Throughout the present disclosure, the term "*at least one performance parameter*" as used herein refers to one or more parameters that are associated with a performance of the first user, such as in one or more physical activities (for example, running, skiing, weightlifting, powerlifting, long jump, high jump, pole vault, javelin, and the like). It will be appreciated that a given performance parameter (i.e., a specific performance parameter from amongst the at least one performance parameter) is associated with the performance of a given first user in a given physical activity. In other words, the given performance parameter is an indicator that enables to measure how well the first user is performing in the given physical activity. Optionally, the given physical activity is performed during a main competition or during a training emulation.

Optionally, the at least one performance parameter comprises at least one of: a time of completion of at least one physical activity performed by the first user, a speed of the at least one physical activity, a weight lifted by the first user during the at least one physical activity, a distance covered during the at least one physical activity, a height covered during the at least one physical activity, and/or a maximal aerobic capacity of the first user during the at least one physical activity. In this regard, the at least one performance parameter is related to a specific attribute or property that enables to evaluate the performance of the first user in terms of a measurable quantity. Herein, a lower value for the time of completion of the at least one physical activity indicates a higher level of the performance of the first user in the at least one physical activity, where the at least one physical activity may be running, skiing, walking, and the like. As an example, what is the time for running 800 meters. Likewise, a higher value of the speed of the at least one physical activity indicates the higher level of the performance of the first user in the at least one physical activity, where the at least one physical activity may be running, cycling, swimming, and the like. For example how fast (m/s) the first user is able to cycle. Likewise, the higher value of the weight lifted by the first user during the at least one physical activity indicates the higher level of the performance of the first user in the at least one physical activity, where the at least one physical activity may be weightlifting, powerlifting, and the like. For example, how many kg the user can lift. Likewise, the higher value of the distance covered during the at least one physical activity indicates the higher level of the performance of the first user in the at least one physical activity, where the at least one physical activity may be long jump, ski jump, javelin throw, and the like. Likewise, the higher value of the height covered during the at least one physical activity indicates the higher level of the performance of the first user in the at least one physical activity, where the at least one physical activity may be pole vault, high jump, and the like. Likewise, the higher value of the a maximal aerobic capacity (i.e., a maximum amount of oxygen that is consumed by the first user during the at least one physical activity) of the first user during the at least one physical activity indicates the higher level of the performance of the first user in the at least one physical activity, where the at least one physical activity may be cycling, running, and the like. Optionally, the at least one performance parameter comprises a power applied by the first user during the at least one physical activity. Thus, beneficially, the method is suitable to use a wide range of attributes or properties associated with the at least one physical activity as the at least one performance parameter.

Notably, change in the at least one performance parameter is indicative of a change in the performance of the first user in the at least one physical activity. Subsequently, by knowing the future value of the at least one performance parameter of the first user (i.e., the value of the at least one performance parameter in a future instance of time), the performance of the first user in the future instance of time can be known. In this regard, the method enables in estimating the future value of the at least one performance parameter to determine the performance of the first user in the at least one physical activity in the future instance of time.

The term "*first user*" as used herein refers to a person, such as an athlete, for whom the at least one performance parameter is required to be measured. It will be appreciated that the first user may be selected from amongst the first set of users which is further selected from the plurality of users. In an example, the plurality of users may be athletics, the first set of users may be runners (that are selected on a basis of top performance in the performance parameter of running), and the first user is a top-performing running shortlisted to be in a team.

The method comprises measuring, for the first user, a first set of values of at least one molecular biomarker and a first set of corresponding values of the at least one performance parameter. Throughout the present disclosure, the term "*molecular biomarker*" as used herein refers to a measurable indicator in a body chemistry such as hormones of the first user that indicates a level of a certain attribute related to a disease or physiological condition of the first user.

In general the term "*molecular biomarker*" can refer to that measurable indicator in the body of the first user which is in form of a hormone in the body of the first user. For example, the at least one molecular biomarker is a level of testosterone in the body of the first user. Alternatively, the at least one molecular biomarker can be cortisol level or for example ratio between testosterone to cortisol level. Notably, the first set of values of the at least one molecular biomarker are determined from a first set of biological samples collected from the first user. It will be appreciated that, a given value of the at least one molecular biomarker is measured for a time instance at which the biological sample is collected from the first user. Optionally, the first set of biological samples are in the form of saliva that is collected directly from a mouth cavity of the first user using a salivary slab or using known techniques that are associated with electrochemical sensors. Alternatively, the first set of biological samples are in the form of at least one of: sweat, urine, blood, plasma, intracellular fluids, extracellular fluids, and the like.

To differentiate from the molecular biomarker, an other type of biomarker "functional biomarker" (value) can be measured. Throughout the present disclosure, the term "*functional biomarker*" refers to that measurable electrical or movement related indicator in the body of the first user. For example, the at least one functional biomarker is a value of heart rate variability of the first user. Notably, the at least one functional biomarker is measured in real-time using sensors such as a heart rate sensor, an accelerometer, and the like.

In general biomarkers can be thus molecular biomarkers or functional biomarkers. Those are technically different as the molecular biomarkers related to body chemistry and those are measured with totally different type of measurement devices than functional biomarkers.

Optionally, at least one molecular biomarker is at least one of: testosterone, cortisol, insulin, growth hormone, corticotropin-releasing hormone (CRH), adrenocorticotropic hormone (ACTH), androstenedione, Insulin-like growth factor 1 (IGF-1), nerve growth factor (NGF), interleukin 6 (IL-6), c-reactive protein (CRP). In this regard the aforementioned molecular biomarkers are different types of hormones that indicate the condition of the body of the first user and may be used for diagnosing diseases, monitoring a disease progression, assessing effectiveness of treatments, and predicting outcomes of treatments. Subsequently, the at least one molecular biomarker is at least one hormone. A technical effect of the at least one molecular biomarker being the at least one hormone is that a correlation is established between levels of the at least one hormone in a body of the first user, and the at least one performance parameter of the first user. Thus, advantageously, the method is suitable to be used for measuring a wide range of at least one molecular biomarker to determine an accurate and precise condition of the body of the first user. For example, the first set of values of the at least one molecular biomarker comprises the values of the IGF-1 measured to be 90 nanograms/milliliter (ng/ml), 120 ng/ml, and 140ng/ml measured at three respective time instances.

Optionally, each value in the first set of values of at least one molecular biomarker is a value corresponding to a ratio of amount of testosterone to amount of cortisol (T/C). In this regard, the T/C indicates a balance between anabolic (i.e., building up) processes, and catabolic (i.e., breaking down) processes in the first user's body. Notably, the T/C is referred to as anabolic quotient. It will be appreciated that, although the amount of testosterone is measured in picomole/liter (pmol/l) and the amount of cortisol is measured in nanomole/liter (nmol/l), but the difference in the measurement units of the amount of testosterone and the amount of cortisol is ignored while calculating the anabolic quotient. For example, the amount of testosterone is measured to be 620 pmol/l and the amount of cortisol is measured to be 8 nmol/l, and subsequently, the T/C is measured to be 77.5. Moreover, a higher value of the T/C indicates to a favorable balance between the anabolic and the catabolic processes, and thus, results in a better physical performance, muscle growth, and recovery for the first user. Thus, advantageously, the T/C is suitable as the at least one molecular biomarker for the first user as it provides information regarding the physiological conditions that are relevant to performing of the at least one physical activity by the first user. In other words, the first set of values of the at least one molecular biomarker can be considered as combination of two separate molecular biomarkers i.e. a first set of values is a set of values calculated from a first molecular biomarker and a second molecular biomarker. The first molecular biomarker would be testosterone and the second molecular biomarker cortisol value. Calculation would refer dividing the testosterone value with cortisol value.

Notably, a given corresponding value (i.e., a specific corresponding value from amongst the first set of corresponding values) of the at least one performance parameter is a value of the at least one performance parameter that is measured for performing the at least one physical activity after a predefined period of time after a time instance at which the given value of the at least one molecular biomarkeris measured. Subsequently, the first set of values of at least one molecular biomarker and a first set of corresponding values of the at least one performance parameter are measured. For example, for the value of the at least one at least one molecular biomarker that is measured to be 77.5 (i.e., the T/C), the corresponding value of the at least one parameter is measured to be 12 meters per second (m/s) (speed of running) (i.e., speed of the at least one physical activity by the first user).

Optionally, each value of the at least one molecular biomarker in the first set is measured at a given first moment of time, and wherein each corresponding value of the at least one performance parameter is measured at a given second moment of time, wherein the given second moment of time is after the given first moment of time. Throughout the present disclosure, the terms "*first moment of time*" and "*second moment of time*" refer respectively to particular moments of time at which a given value of the at least one molecular biomarker is measured and corresponding to which a given value of the at least one performance parameter is measured. It will be appreciated that a time difference of a predefined period of time exists between the given first moment of time and the given second moment of time. Optionally, the given first moment of time is at an evening of a given day at which the given value of the at least one molecular biomarker is measured and the given second moment of time for measuring the given corresponding value of the at least one performance parameter is at a consecutive day following the evening of measurement of the at least one molecular biomarker. For example, if the given value of the at least one molecular biomarker is measured on the evening of a Monday, then the given corresponding value of the at least one performance parameter is measured on a following Tuesday (i.e., the consecutively following day to the evening of the Monday). Thus, beneficially, the values of the at least one molecular biomarker and the corresponding values of the at least one performance parameter are measured in fixed patterns with respect to time to get more accurate and precise results. Moreover, advantageously the time difference of the predefined period of time is used to provide a supplements to the first user to improve achieve better results in the at least one performance parameter that is measured for the user. Time difference between the first movement of time and the second moment of time can be 1, 2, 4, 6, 8, 10, 12, 14, 16, 18, 20, 22 or 24 hours. The first moment of time has been found out to correlate best if it is selected just before going to sleep, on previous night before the associated measuring of the at least one performance parameter. Also, when using the derived prediction function a prediction request value (i.e. test sample of the molecular biomarker) should be taken at same moment of time as has been used when creating the function. As an example, if the function is generated using measurements done just before going to sleep (say at 21.30 PM) then the prediction request value should be also taken at the same time.

Moreover, the method comprises determining a first correlation between the first set of values of the at least one molecular biomarker and the first set of corresponding values of the at least one performance parameter. Throughout the present disclosure, the term "*first correlation*" refers to a specific relation between the first set of values of the at least one molecular biomarkerto the first set of corresponding values of the at least one performance parameter. Notably, the first correlation is determined to know how the first set of values of the at least one molecular biomarker influences the first set of corresponding values of the at least one performance parameter for the first user. Optionally, the first set of values for a said at least one molecular biomarker are directly proportional or inversely proportional to the first set of corresponding values of a said performance parameter. For example, the first correlation is of a value of 0.734 between the speed of running 800 m and the T/C. Example correlation is a linear correlation (i.e. fitting the measured the first set of values (of for example T/C) and measured performance parameter (for example speed of runner in m/s) to find linear correlation between the two.). Alternative correlation could be inverse correlation.

Furthermore, the method comprises using the determined first correlation to generate a first performance prediction function for the first user that indicates the at least one performance parameter as a function of the at least one molecular biomarker. Throughout the present disclosure, the term "*first performance prediction function*" is a mathematical function that uses the value of the at least one molecular biomarker to indicate the corresponding value of the at least one performance parameter for the first user. It will be appreciated that, optionally, a historical medical data of the first user may also be used in generating the first performance prediction parameter. In other words, the first performance prediction indicates a said value of the at least one performance parameter for a said value of the at least one molecular biomarker for the first user, without the first user performing the at least one physical activity. It will be appreciated that, the first performance prediction function is generated using the determined first correlation that indicates how the first set of values of the at least one molecular biomarker influences the first set of corresponding values of the at least one performance parameter for the first user. Notably, the generated first performance prediction function is used to predict the given value of the at least one performance parameter at a moment of time in future. Optionally, the first performance prediction function is one of: a polynomial function, an exponential function, and the like. For example, the first performance prediction function is generated to v(T/C) = 8.23*(T/C) + 5.63, where v(T/C) refers to the speed of running 800 m as a function of T/C. Other functions such as polynomial, exponential etc. can be envisioned. Furthermore, the function can be based on statistical analysis of the date i.e., machine learning fitted model to the measured first set of values of at least one molecular biomarker and the first set of corresponding values of the he at least one performance parameter.

Optionally, the first performance prediction function is generated using the first set of values of the at least one molecular biomarker and the first set of corresponding values of the at least one performance parameter. In this regard, the first set of values of the at least one molecular biomarker and the first set of corresponding values of the at least one performance parameter are used in a mathematical algorithm of function fitting to generate the first performance prediction function. Thus, advantageously, the first performance prediction is able to estimate the future value of the at least one performance parameter based on the physiological conditions (i.e., the least one molecular biomarker) of the first user.

Furthermore, the method comprises measuring, for the first user, a prediction request value of the at least one molecular biomarker. Throughout the present disclosure, the term "*prediction request value of the at least one molecular biomarker*" refers to a value of the biomarker measured after the first performance prediction function is generated. Herein, the prediction request value of the biomarker is used as an input for the first performance prediction function. For example, the prediction request value of the at least one molecular biomarker is measured to be the T/C value of 65. An example of using the request value of the at least one molecular biomarker is to measure the molecular biomarker one day before competition. The measured prediction request value is used as input to the prediction function. The prediction function will provide predicted performance for the next day event. As an example, provide prediction that time for 800 m run will be 2 minutes 5.42 secs. Furthermore, the prediction request value (i.e. sample value) should be preferably taken at a same moment of time as the measured first set of values were taken. The moment of time can refer to time of the day or hours before the at least one performance.

Furthermore, the method comprises using the first performance prediction function to estimate the future value of the at least one performance parameter, based on the prediction request value of the at least one molecular biomarker. In this regard, the prediction request value of the at least one molecular biomarker is used as an input to solve an equation of the first performance prediction function. Subsequently, a result from solving the equation of the first performance prediction function is the estimated future value of the at least one performance parameter. For example, the first performance prediction function estimates the future value of the at least one performance parameter to be 6.16m/s for 800m run, based on the prediction request value of the at least one molecular biomarker that is the T/C value is 65.

The present disclosure also relates to the system and to the computer program product as described above. Various embodiments and variants disclosed above, with respect to the aforementioned method, apply *mutatis mutandis* to the system and to the computer program product.

The term "*measurement arrangement*" as used herein refers to an arrangement of components that is used to measure a numerical quantity of the at least one molecular biomarker in the body of the first user. Optionally, measurement arrangement is a form of electrochemical sensor that is suitable to sense the given value of the at least one molecular biomarker from the given biological sample. As an example, the measurement arrangement can be biological sample collection device. One example of measurement arrangement is a device which is configured to measure both value of testosterone and cortisol and provide ratio of the two as output. Benefit of using such device is elimination of measurement noise (as the measurement noise might be same for both values of T and C i.e. those cancel out when output is T/C).

The term "*measurement device*" as used herein refers to device that is used to measure a numerical value of the at least one performance parameter for the first user for the first type of the physical activity. Notably, the measurement device is in form of an electronics device that is capable of measuring at least one of: a distance covered, a time of completion, a speed, a weight lifted, and the like. It will be appreciated that, a type of measurement device that is used depends on the at least one performance parameter that is measured. Optionally, the measurement device measures more than one performance parameters. For example, the measurement device is a stopwatch that measures the time of completion of a said physical activity, the distance covered in performing the said physical activity, and the speed of performing the said physical activity. Alternatively, the measurement device is an accelerometer.

The term "*server*" as used herein refers to a hardware, software, firmware or a combination of these, suitable for controlling the operation of the system. The server is communicably coupled to the measurement arrangement and the measurement device. Examples of the server include, but are not limited to, a microprocessor, a microcontroller, a complex instruction set computing (CISC) microprocessor, a reduced instruction set (RISC) microprocessor, a very long instruction word (VLIW) microprocessor, or any other type of processing circuit. Furthermore, the server may refer to one or more individual servers, serving devices and various elements associated with a serving device that may be shared by other serving devices. Additionally, one or more individual servers, serving devices and elements are arranged in various architectures for responding to and processing the instructions that drive the system.

The server is configured to receive the first set of values of the at least one molecular biomarker and the first set of corresponding values of the at least one performance parameter; and generate, using the received first set of values of the at least one molecular biomarker and the first set of corresponding values of the at least one performance parameter, a first performance prediction function for the first user that indicates the at least one performance parameter as a function of the at least one molecular biomarker

Optionally, the server is configured to receive a prediction request value of the at least one molecular biomarker for the first user; and use the first performance prediction function to estimate the future value of the at least one performance parameter, based on the prediction request value of the at least one molecular biomarker. In this regard, the prediction request value of the at least one molecular biomarker is received for the first user by collecting a biological sample from the first user and subsequently, measuring a value of the at least one molecular biomarker in the collected biological sample which acts as the prediction request value of the at least one molecular biomarker Thus, advantageously, the future value of the at least one performance parameter is estimated accurately according to a physical condition of the first user's body that is indicated in the prediction request value of the at least one molecular biomarker for the first user.

Optionally, the system comprises an output interface to output the future value of the at least one performance parameter. The term "*output interface*" as used herein refers to a graphical user interface that is configured to display the future value of the at least one performance parameter as the output to the first user. Optionally, the output interface is implemented as a display screen that is arranged on a side or a face of the system. Thus, advantageously, the first user is able to view and track the future value of the at least one performance parameter in a visually immersive and interactive manner.

The present disclosure also relates to the method for selecting a first set of users from amongst a plurality of users as described above. Various embodiments and variants disclosed above, with respect to the aforementioned method of the first aspect, apply *mutatis mutandis* to the method of the third aspect.

Throughout the present disclosure, the term "*plurality of users*" refers to more than one users whose respective performances in performing the first type of physical activity is tracked. Throughout the present disclosure, the term "*first set of users*" refers to group of users from amongst the plurality of users, where the first set of users are to be determined based on a criteria of top performance.

The method for selecting a first set of users from amongst a plurality of users comprises generating respective performance prediction functions for the plurality of users. In this regard, the respective performance prediction function is generated for each of the respective user based on a respective set of values of at least one molecular biomarker and respective corresponding set of values of the at least one performance parameter for the respective user. The performance prediction functions are generated using methods discussed above in conjunction to other embodiments.

Moreover, the method comprises receiving respective prediction request values of at least one molecular biomarker for the plurality of users. In this regard, the respective prediction request value of the at least one molecular biomarkeris received separately for the respective user.

Furthermore, the method comprises using, the respective prediction request values of the at least one molecular biomarker as respective inputs to the respective performance prediction functions for the plurality of users, for estimating respective future values of at least one performance parameter for the plurality of users. In this regard, the respective future value of the at least one performance parameter is estimated separately for the respective user, using the respective prediction request value of the at least one molecular biomarkeras the respective input to the respective performance prediction function of the respective user.

Furthermore, the method comprises selecting the first set of users from amongst the plurality of users, based on the respective future values of the at least one performance parameter for the plurality of the users, wherein the first set of users comprise users having N highest values out of the future values of the at least one performance parameter, wherein the first set of users comprises N users. In this regard, the respective futures values of the at least one performance parameter for the plurality of users, are sorted in a descending order and then, analyzed to identify the N number of highest values from amongst the future values of the at least one performance parameter. Notably, the respective N users for whom the N highest values are identified, belong to a category of a top performers from amongst the plurality of users. Subsequently, the N users are selected as the first set of users, and thus, the first set of users comprises N users. Thus, advantageously, the first set of users are selected from amongst the plurality of users, based on who the top performers are from the amongst the plurality of users. This can be used for example when selecting a team for 4x400 meter relay. As an example, the plurality of users are 6 runners. A first runner having his season best 61 sec, a second user having season best 59 sec, a third user having season best 55 sec, a fourth user having season best 66 sec, a fifth user having season best 54 sec and a sixth user having season best 55 sec. If selection for runners of the relay is done using the season best times, then the fifth user (54 sec) the sixth user (55 sec), the third user (55 sec) and the second user (59 sec) would be selected. Based on embodiments a performance prediction function for each of the plurality of users (6 in this example) is generated. A day before (previous evening for example) a prediction request values of at least one molecular biomarkeris received (measured) from each of the plurality of users. For example, T/C for users is received. This is used to estimate future value of the at least one performance. In this example predicted speeds would be for the first 58 sec, the second user 65 sec, the third user 54 sec, the fourth user 66 sec, the fifth user 66 sec and the sixth user 57 sec. I.e these predicted future values are used to select the first set of user (4 predicted best ones) from the plurality of user (all 6 runners) based on those having N highest values. The highest now refers to fastest speed (the actual value is lowest but from performance point of view the values are highest performance values i.e. performance is higher smaller the time is). In present example the relay team would be the third user (54 sec), the sixth user (57 sec), the first user (58 sec). I.e the fifth and the second user would be dropped out from the team. This enables to select participants using predicted future performance instead of statistical past history data.

Optionally, a given performance prediction function is generated according to the aforementioned method of the first aspect. Advantageously, the given performance prediction function estimates the future value of the at least one performance parameter based on a physical condition of a body of the given user that is indicated in the given prediction request value of the at least one molecular biomarker for the given user.

As a summary a method for estimating a future value of at least one performance parameter of a first user is provided. The future value can refer to how fast the first user runs in a next coming event (for example tomorrow or later during the day). This is achieved by measuring a set of data values, which the data values are at least one molecular biomarker and corresponding value of at least one performance value. For example, measuring weekly on Monday evenings testosterone value and cortisol value and dividing testosterone value with cortisol value. Then conducting performance on Tuesday noon (for example running 800 meters), wherein the performance is conducted with "all in principle" i.e., as fast as possible. These measurement pairs are collected and correlation between the measured molecular biomarkesr and the measured performance parameters is found. This correlation is used to generate a prediction function. (Correlation and function generation can be also a single step i.e. the values can be used to determine the function without correlation step).

After generation of the prediction function, it can be used. In practice now the first user wants to know performance of tomorrow. The person will measure same molecular biomarker as used for generating the prediction function (T/C in practical example). This is referred as a prediction request value. This prediction request value is used as input to the performance prediction function. The function will output future performance which can be displayed for the user for example in smart phone.

In alternative embodiment of the present disclosure provides a method for estimating a future value of at least one performance parameter of a first user, the method comprising:
measuring, for the first user, a first set of values of at least one biomarker and a first set of corresponding values of the at least one performance parameter;
determining a first correlation between the first set of values of the at least one biomarker and the first set of corresponding values of the at least one performance parameter;
using the determined first correlation to generate a first performance prediction function for the first user that indicates the at least one performance parameter as a function of the at least one biomarker;
measuring, for the first user, a prediction request value of the at least one biomarker; and
using the first performance prediction function to estimate the future value of the at least one performance parameter, based on the prediction request value of the at least one biomarker. Preferably in the alternative embodiment the biomarker is molecular biomarker.

### DETAILED DESCRIPTION OF THE DRAWINGS

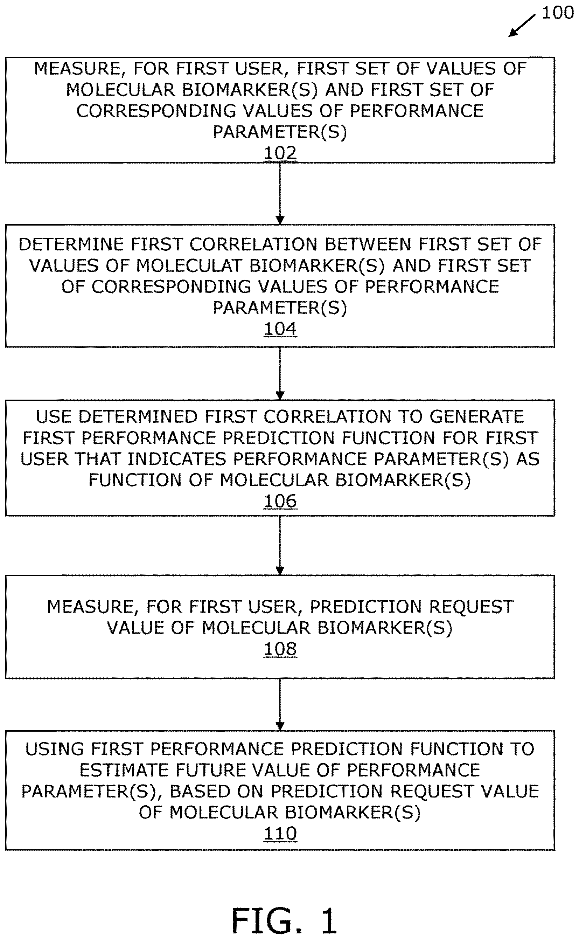

Referring to FIG. 1, illustrated is a flowchart **100** are steps of a method for estimating a future value of at least one performance parameter of a first user, in accordance with an embodiment of the present disclosure. At step **102,** a first set of values of at least one molecular biomarker and a first set of corresponding values of the at least one performance parameter is measured for the first user. At step **104,** a first correlation between the first set of values of the at least one molecular biomarker and the first set of corresponding values of the at least one performance parameter is determined. At step **106,** the determined first correlation is used to generate a first performance prediction function for the first user that indicates the at least one performance parameter as a function of the at least one molecular biomarker. At step **108,** a prediction request value of the at least one molecular biomarker is measured for the first user. At step **110,** the first performance prediction function is used to estimate the future value of the at least one performance parameter, based on the prediction request value of the at least one molecular biomarker.

The aforementioned steps **102, 104, 106, 108,** and **110** are only illustrative and other alternatives can also be provided where one or more steps are added, one or more steps are removed, or one or more steps are provided in a different sequence without departing from the scope of the claims herein.

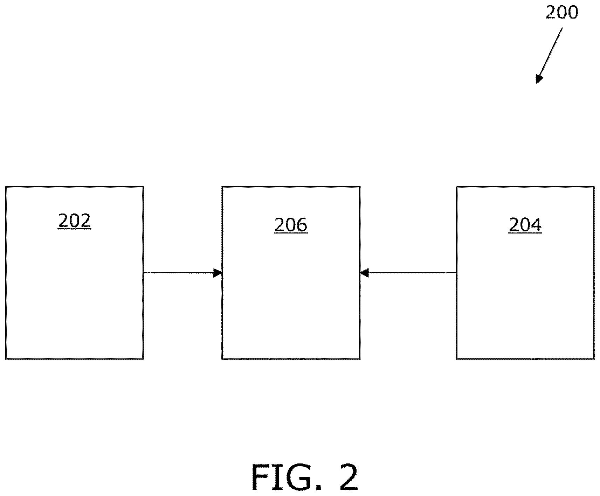

Referring to FIG. 2, illustrated is a block diagram of a system **200** for estimating a future value of at least one performance parameter of a first user, in accordance with an embodiment of the present disclosure. The system **200** comprises a measurement arrangement **202** and a measurement device **204.** Moreover, the system **200** comprises a server **206,** where the server **206** is communicably coupled to the measurement arrangement **202** and the measurement device **204.**

It may be understood by a person skilled in the art that the FIG. 1 includes a simplified architecture of the system **200** for sake of clarity, which should not unduly limit the scope of the claims herein. It is to be understood that the specific implementation of the system **200** is provided as an example and is not to be construed as limiting it to specific numbers or types of servers and data repositories. The person skilled in the art will recognize many variations, alternatives, and modifications of embodiments of the present disclosure.

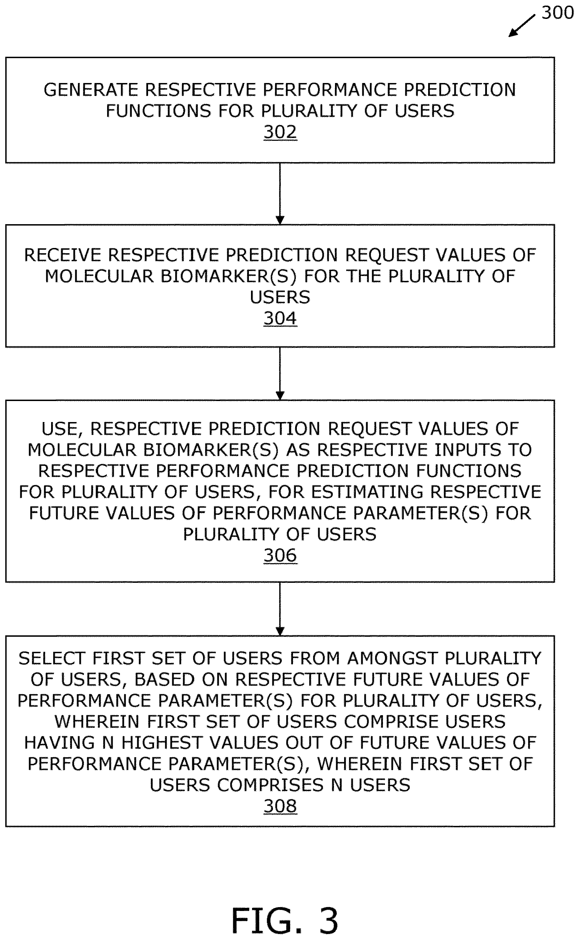

Referring to FIG. 3, illustrated is a flowchart **300** depicting steps of a method for selecting a first set of users from amongst a plurality of users, in accordance with an embodiment of the present disclosure. At step **302,** respective performance prediction functions are generated for the plurality of users. At step **304,** respective prediction request values of at least one molecular biomarker are received for the plurality of users. At step **306,** the respective prediction request values of the at least one molecular biomarker are used as respective inputs to the respective performance prediction functions for the plurality of users, for estimating respective future values of at least one performance parameter for the plurality of users. At step **308,** the first set of users are selected from amongst the plurality of users, based on the respective future values of the at least one performance parameter for the plurality of the users, wherein the first set of users comprise users having N highest values out of the future values of the at least one performance parameter, wherein the first set of users comprises N users.

The aforementioned steps **302, 304, 306,** and **308** are only illustrative and other alternatives can also be provided where one or more steps are added, one or more steps are removed, or one or more steps are provided in a different sequence without departing from the scope of the claims herein.

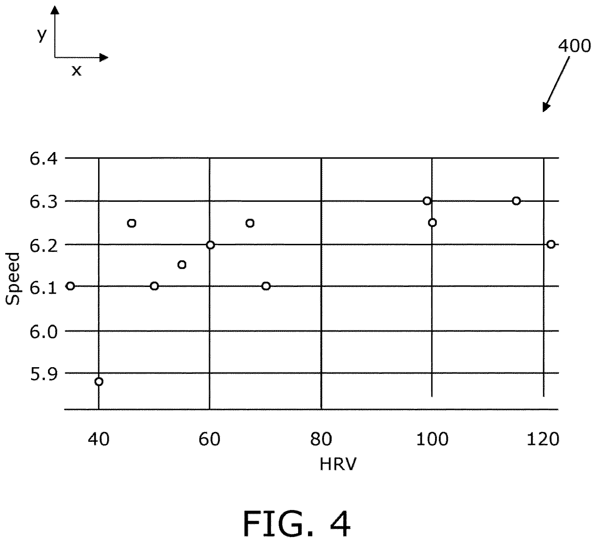

Referring to FIGs. 4-9, illustrated are graphical representations of a respective performance parameter with respect to a respective molecular or functional biomarker, in accordance with various embodiment of the present disclosure.

As a first experiment (EXPERIMENT I) we conducted a series of tests with a top athletic in the field of running (specialised to 400 - 1500 meters).

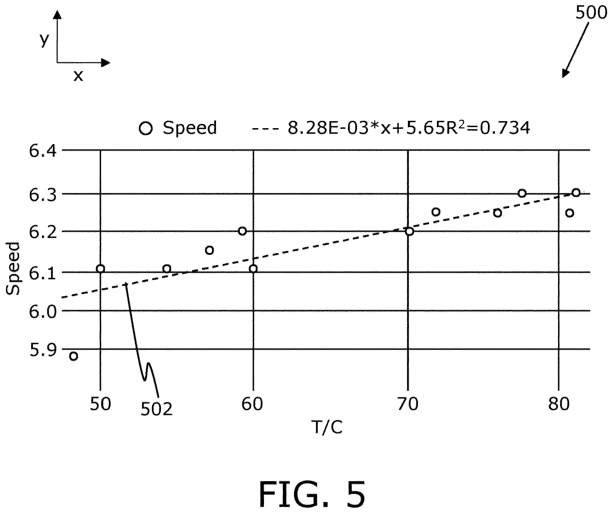

As shown in FIG. 4, y-axis of the graphical representation **400** depicts a functional biomarker which is a heart rate variability (HRV) of the user (said top athletic). Moreover, x-axis of the graphical representation **400** depicts the performance parameter which a speed of performing a physical activity. It can be seen that there is little or no correlation between HRV and the speed, confirming the technical problem that for example HRV might provide unreliable information. As shown in FIG. 5, x-axis of the graphical representation **500** depicts a molecular biomarker which is a ratio of amount of testosterone to amount of cortisol (T/C) for the user. Moreover, y-axis of the graphical representation **500** depicts the performance parameter which is a speed (m/s) of performing a physical activity. Speed was calculated by dividing 800 meters with time it took to run the 800 meters i.e., the speed is in this experiment average speed. Furthermore, a line **502** in the graphical representation **500** depicts the speed of performing the physical activity as a function of the ratio of the amount of testosterone to the amount of cortisol for the user. A clear correlation can be seen for T/C and speed (m/s) value. Indeed, R2 value is 0.734 indicating good linear fit. I.e. according to the experiment we are able to generate a first performance prediction function for the first user. This function can be used to predict a future value of at least one performance parameter. The generated function y=8.28*10-3x+5.65 provides, for example when T/C is 100 (as measured previous day) = 0.828 + 5.65 = 6.48 m/s i.e., time for 800 m will be 2 minutes and 3 sec.

A second experiment (EXPERIMENT II) a bodybuilder performed repetition maximum value tests. In said test 1RM values were measured. The bodybuilder, who tracked his pectoralis major muscle performance as a set performed into voluntary exhaustion. In strength sports, it is easy to execute a maximum test like this, because it's affected into total training volume is not marked as performing endurance test on maximal performance level. Test was done performing one set into a failure. The used weight was approx. 100 kg. Subject performed one set with as many repetitions as he could. Repetitions and weight was documented and the 1RM value was assessed using Fleck and Kraemer formula (1RM = weight - [(1.0278) - (0.0278 x repetitions)] (kg).

The molecular biomarker value of ratio between T/C was measured in the evening preceding the test day. Following results were observed. It seems surprisingly that there is correlation between T/C value (x-axis) and 1RM values (y-axis). Indeed for the person in question 1RM as function of T/C is 1RM(T/C) = 0.1575 x (T/C) + 114.67.

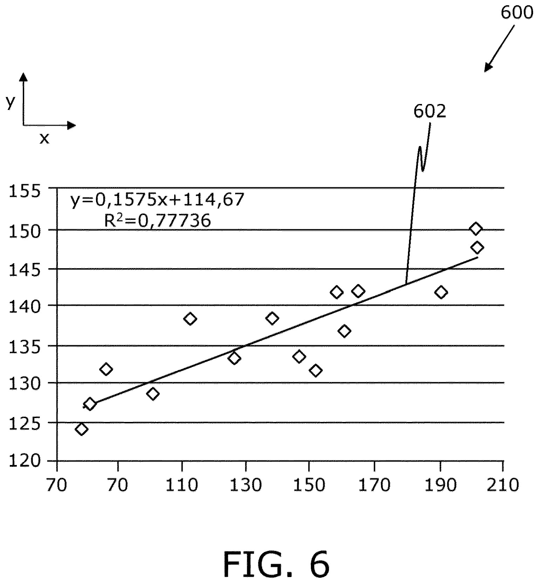

There is good liner correlation as indicated by R2 value of 0.77736. As shown in FIG. 6, x-axis of the graphical representation **600** depicts the molecular biomarker which is a ratio of amount of testosterone to amount of cortisol for the user (bodybuilder). Moreover, y-axis of the graphical representation **600** depicts the performance parameter which is a repetition maximum value of lifting a weight (1RM value). Furthermore, a line **602** in the graphical representation **600** depicts the repetition maximum value of lifting the weight as a function of the ratio of the amount of testosterone to the amount of cortisol for the user.

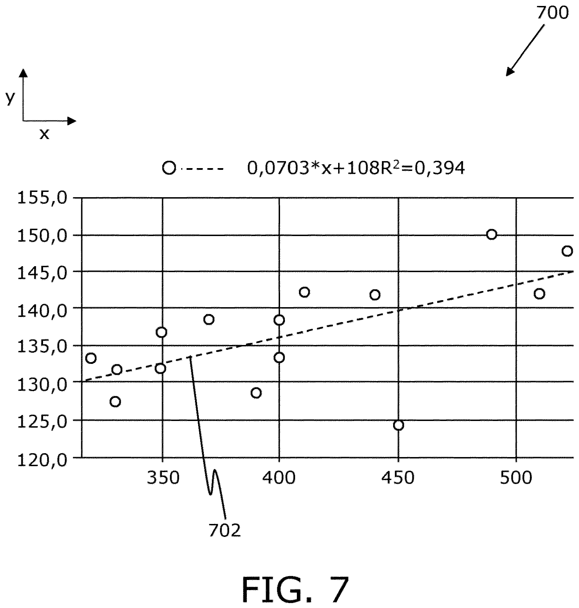

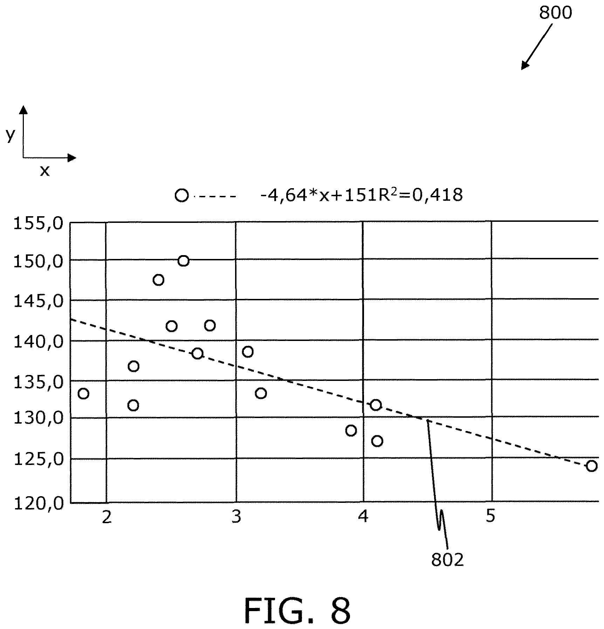

As shown in FIG. 7, x-axis of the graphical representation **700** depicts the molecular biomarker which is an amount of testosterone for the user. Moreover, y-axis of the graphical representation **700** depicts the performance parameter which is a repetition maximum value of lifting a weight. Furthermore, a line **702** in the graphical representation **700** depicts the repetition maximum value of lifting the weight as a function of the amount of testosterone for the user. We can see in this example that there the correlation value R2 = 0.394 which is significantly lower than when using T/C. Furthermore in FIG. 8, x-axis of the graphical representation **800** depicts the molecular biomarker which is an amount of cortisol for a user. Moreover, y-axis of the graphical representation **800** depicts the performance parameter which is a repetition maximum value of lifting a weight. Furthermore, a line **802** in the graphical representation **800** depicts the repetition maximum value of lifting the weight as a function of the amount of cortisol for the user. For this we can also see that correlation value R2 = 0.418 which is lower than when using T/C further demonstrating that if a molecular biomarker value of T/C is used a reliable performance function can be derived. To further demonstrate the technical effect in FIG. 8, x-axis of the graphical representation **800** depicts the molecular biomarker which is a ratio of amount of testosterone to amount of cortisol for a user. Indeed as conclusion of experiment II we can see that using T/C we can generate prediction function with good correlation (linear). If we would use only T or C correlation would be not as good.

Moreover, y-axis of the graphical representation **900** depicts the performance parameter which is an energy used to perform the physical activity (for the same body builder). Furthermore, a line **902** in the graphical representation **900** depicts the energy used to perform the physical activity as a function of the ratio of the amount of testosterone to the amount of cortisol for the user. A clear correlation can be observed. The y-axis value is derived from 1RM tests above by taking in consideration how high (meter) the weights were lifted and how fast (in sec).

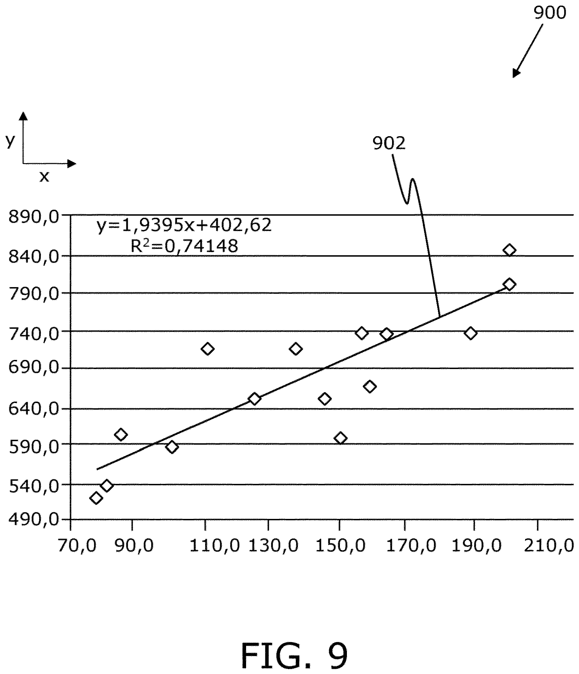

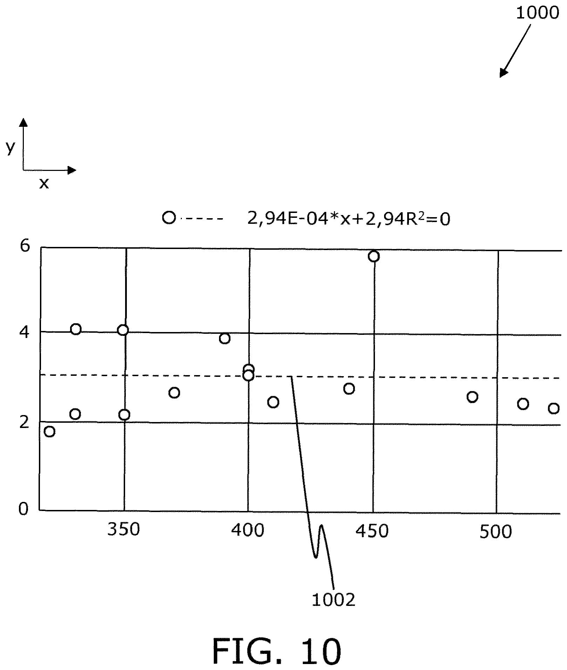

Referring to FIG. 10, illustrated is a graphical representation **1000** of a first biomarker with respect to a second biomarker, in accordance with an embodiment of the present disclosure for the bodybuilder of the example. As shown in FIG. 9, x-axis of the graphical representation **1000** depicts the first biomarker as a molecular biomarker which is an amount of testosterone for a user. Moreover, y-axis of the graphical representation **1000** depicts the second biomarker as another molecular biomarker which is an amount of cortisol for the user. Furthermore, a line **1002** in the graphical representation **1000** depicts the amount of testosterone for the user as a function of the amount of cortisol for the user. We can see, in this experiment, there is no correlation between the values.

Modifications to embodiments of the present disclosure described in the foregoing are possible without departing from the scope of the present disclosure as defined by the accompanying claims. Expressions such as "including", "comprising", "incorporating", "have", "is" used to describe and claim the present disclosure are intended to be construed in a non-exclusive manner, namely allowing for items, components or elements not explicitly described also to be present. Reference to the singular is also to be construed to relate to the plural.

## Claims

1. A method for estimating a future value of at least one performance parameter of a first user, the method comprising:
measuring, for the first user, a first set of values of at least one molecular biomarker and a first set of corresponding values of the at least one performance parameter;
determining a first correlation between the first set of values of the at least one molecular biomarker and the first set of corresponding values of the at least one performance parameter;
using the determined first correlation to generate a first performance prediction function for the first user that indicates the at least one performance parameter as a function of the at least one molecular biomarker;
measuring, for the first user, a prediction request value of the at least one molecular biomarker; and
using the first performance prediction function to estimate the future value of the at least one performance parameter, based on the prediction request value of the at least one molecular biomarker.

2. The method according to claim 1, wherein the at least one molecular biomarker is at least one of: testosterone, cortisol, insulin, growth hormone, corticotropin-releasing hormone (CRH), adrenocorticotropic hormone (ACTH), androstenedione, Insulin-like growth factor 1 (IGF-1), nerve growth factor (NGF), interleukin 6 (IL-6), c-reactive protein (CRP).

3. The method according to claim 1 or 2, wherein each value in the first set of values of the at least one molecular biomarker is a value corresponding to a ratio of amount of testosterone to amount of cortisol.

4. The method according to any of the preceding claims, wherein the at least one performance parameter comprises at least one of: a time of completion of at least one physical activity performed by the first user, a speed of the at least one physical activity, a weight lifted by the first user during the at least one physical activity, a distance covered during the at least one physical activity, a height covered during the at least one physical activity, and/or a maximal aerobic capacity of the first user during the at least one physical activity.

5. The method according to any of the preceding claims, wherein each value of the at least one molecular biomarker in the first set is measured at a given first moment of time, and wherein each corresponding value of the at least one performance parameter is measured at a given second moment of time, wherein the given second moment of time is after the given first moment of time.

6. The method according to any of the preceding claims, wherein the first performance prediction function is generated using the first set of values of the at least one molecular biomarker and the first set of corresponding values of the at least one performance parameter.

7. A system (100) for estimating a future value of at least one performance parameter of a first user, the system comprising
a measurement arrangement (102) for measuring a first set of values of at least one molecular biomarker for a first user;
a measurement device (104) that is to be employed for measuring a first set of corresponding values of the at least one performance parameter for the first user for a first type of physical activity; and
a server (106) configured to:
receive the first set of values of the at least one molecular biomarker and the first set of corresponding values of the at least one performance parameter; and
generate, using the received first set of values of the at least one molecular biomarker and the first set of corresponding values of the at least one performance parameter, a first performance prediction function for the first user that indicates the at least one performance parameter as a function of the at least one molecular biomarker.

8. The system (100) according to claim 7, wherein the server (106) is configured to:
receive a prediction request value of the at least one molecular biomarker for the first user; and
use the first performance prediction function to estimate the future value of the at least one performance parameter, based on the prediction request value of the at least one molecular biomarker.

9. The system (100) according to claim 7 or 8, wherein the system comprises an output interface to output the future value of the at least one performance parameter.

10. A method for selecting a first set of users from amongst a plurality of users, the method comprising:
generating respective performance prediction functions for the plurality of users;
receiving respective prediction request values of at least one molecular biomarker for the plurality of users;
using, the respective prediction request values of the at least one molecular biomarker as respective inputs to the respective performance prediction functions for the plurality of users, for estimating respective future values of at least one performance parameter for the plurality of users; and
selecting the first set of users from amongst the plurality of users, based on the respective future values of the at least one performance parameter for the plurality of the users, wherein the first set of users comprise users having N highest values out of the future values of the at least one performance parameter, wherein the first set of users comprises N users.

11. The method according to claim 10, wherein a given performance prediction function is generated according to any of claims 1-6.
